(19) Europäisches Patentamt
European Patent Office
Office européen des brevets

(11) **EP 2 553 082 B1**

(12) **EUROPÄISCHE PATENTSCHRIFT**

(45) Veröffentlichungstag und Bekanntmachung des Hinweises auf die Patenterteilung:
**11.03.2015 Patentblatt 2015/11**

(21) Anmeldenummer: **11711338.1**

(22) Anmeldetag: **30.03.2011**

(51) Int Cl.:
***C12M 1/107*** *(2006.01)*

(86) Internationale Anmeldenummer:
**PCT/EP2011/054911**

(87) Internationale Veröffentlichungsnummer:
**WO 2011/121024 (06.10.2011 Gazette 2011/40)**

(54) **VERFAHREN UND VORRICHTUNG ZUR HERSTELLUNG VON BIOGAS**

METHOD AND APPLIANCE FOR PRODUCING BIOGAS

PROCÉDÉ ET DISPOSITIF DE PRODUCTION DE BIOGAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2010 DE 102010014239**

(43) Veröffentlichungstag der Anmeldung:
**06.02.2013 Patentblatt 2013/06**

(73) Patentinhaber: **KSB Aktiengesellschaft
67227 Frankenthal (DE)**

(72) Erfinder:
• **ROSTALSKI, Kai
06217 Merseburg (DE)**
• **SPRINGER, Peer
67141 Neuhofen (DE)**

(56) Entgegenhaltungen:
**EP-A1- 1 394 246       EP-A1- 1 762 607
WO-A2-2009/071294    DE-U1-202007 002 835**

EP 2 553 082 B1

Beschreibung

Die Erfindung betrifft ein Verfahren und eine Vorrichtung zur Herstellung von Biogas aus organischen Stoffen, wobei einem Behälter Substrat mittels eines Aufgabesystems zugeführt wird und in dem Behälter mindestens zwei Rührwerke angeordnet sind, deren Propeller über Antriebe in Rotation versetzt werden, wobei die Propeller in dem Behälter zumeist horizontale Strömungen des Behälterinhalts erzeugen.

Das Verfahren dient der Erzeugung von Biogas aus organischen Stoffen. Der in solchen Anlagen zur Biogaserzeugung eingesetzte Rohstoff wird als Substrat bezeichnet. Das Substrat besteht aus biogen abbaubarer Biomasse wie Gülle, Silage oder Bioabfall. Die zur Biogasproduktion eingesetzten Behälter werden auch als Bioreaktoren oder Fermenter bezeichnet. Im kontinuierlichen Betrieb der Biogasanlagen wird dem Behälter fortlaufend Substrat zugeführt und Biogas sowie Gärrest entnommen. Im Behälter befindliches Substrat wird durch verschiedene Arten von Mikroorganismen umgesetzt. Diese umzusetzende Biomasse wird als Gärsubstrat bezeichnet. Aufgrund des mikrobiellen Abbaus entstehen aus dem Gärsubstrat Methan und Kohlendioxid als Hauptkomponenten des Biogases.

Das zugeführte Substrat wird mit dem Behälterinhalt vermischt. Die Zuführung des Substrates erfolgt meist durch punktförmige Aufgabe mit Hilfe von Fütterungssystemen. Die für eine möglichst hohe Ausbeute an Biogas benötigte Verweilzeit der Biomasse ist entscheidend von der Vermischung des Substrats mit dem Gärsubstrat abhängig. Bei solchen Medien, die überwiegend durch erhöhte Viskositäten gekennzeichnet sind, ist zur Vermischung und/oder Durchmischung eine Umwälzung des Behälterinhalts erforderlich, was in der Regel mit Rührwerken erfolgt.

Im Bereich der anaeroben Bioreaktionstechnik werden bei vielen Anwendungen Fermenter mit einem Höhe zu Durchmesser Verhältnis von größer als 0,5 eingesetzt. Die Vermischung wird dabei meist mit Vertikal-Rührwerken vorgenommen. Dabei befinden sich die Rührwerkspropeller auf einer zentralen von außen angetriebenen Welle. Die Antriebswelle ist vertikal angeordnet und ragt von oben in den Behälter hinein, wobei sie meist parallel zu den Behälterwänden verläuft. Ein solcher Fermenter ist beispielsweise durch die DE 199 47 339 A1 bekannt.

Stattdessen werden bei dem erfindungsgemäßen Verfahren zur Herstellung von Biogas Behälterformen eingesetzt, deren Höhe zu Durchmesser Verhältnis kleiner als 0,5 ist. Der Durchmesser der Behälter liegt vorzugsweise zwischen 16 und 40 Meter. Bei diesen Behälterabmessungen ist der Einsatz eines von außen angetriebenen zentralen Vertikal-Rührwerkes nicht mehr wirtschaftlich. Zur Vermischung des Behälterinhalts kommen vorwiegend in der Randzone des Behälters und darin angeordnete Rührwerke zum Einsatz, die eine zumeist horizontale Strömung des Mediums im Behälter erzeugen. Eine solche Anordnung ist beispielsweise durch die WO 2008/104320 A2 bekannt.

Durch die DE 20 2007 002 835 U1 sind für die Durchmischung des Behälterinhalts mehrere Rührwerke bekannt, wobei zwei übereinander angeordnete Rührwerke einem einzelnen Rührwerk gegenüber angeordnet sind. Für einen hohen Wirkungsgrad des Fermentationsprozesses wird eine möglichst gleichmäßige Biomasseverteilung in der Fermenterflüssigkeit als erforderlich angesehen. Zusätzlich ist eine Füllstandsmessvorrichtung vorgesehen, mit der die Füllhöhe im Behälter erfasst und ein entsprechendes Füllstandsmesssignal als Höhenistwertsignal erzeugt wird. Ein Füllstandsmesssignal wird einer Steuereinrichtung zugeführt, die bei einer erfassten geringeren Füllhöhe einen Höhenstellmotor für das als Tauchmotorrührgerät ausgebildete Rührwerk so ansteuert, dass dieses abgesenkt wird und dessen Rührflügel dadurch weiter vollständig eingetaucht sind.

Die zur Biogaserzeugung eingesetzten Gärsubstrate haben in der Regel strukturviskose Fließeigenschaften. Strukturviskos bedeutet, dass mit zunehmender Schergeschwindigkeit die dynamische Viskosität vom Gärsubstrat abnimmt. Die Viskosität ist somit kein Wert sondern eine Funktion. Zu jeder induzierten Scherrate stellt sich eine dazugehörige Viskosität ein. Damit ist die Viskosität im Behälter örtlich unterschiedlich. Sie hängt von den lokal vorhandenen Scherraten ab. Ursächlich dafür sind die örtlichen Geschwindigkeiten, welche die Strömung im Behälter beeinflussen.

Scherraten werden durch die Bewegung des Propellers eines Rührwerkes erzeugt. In der Umgebung um den Propeller nimmt bei strukturviskosen Gärsubstraten die lokale Viskosität ab. Mit zunehmendem Abstand vom Propeller verringert sich die Schergeschwindigkeit und die Viskosität steigt entsprechend an. Dies führt dazu, dass der Propeller vorwiegend Gärsubstrat aus propellernahen Bereichen ansaugt, in denen das Gärsubstrat eine geringe Viskosität besitzt. Dadurch entstehen propellernahe Bereiche, in welchen das Substrat mit hohen Geschwindigkeiten in einem kleinen Volumen lediglich um den Propeller selbst herum transportiert wird. Diese propellernahen Bereiche werden als Kavernen bezeichnet. Arbeiten Rührwerke lediglich lokal in einer Kaverne findet keine optimale Durchmischung des Behälterinhalts statt, weil sich die Strömungserzeugung auf diese Bereiche beschränkt. Infolgedessen führt dies zur Verringerung des nutzbaren Reaktorvolumens bezogen auf den tatsächlichen Rauminhalt des Bioreaktors. Im Ergebnis wird im kleineren nutzbaren Reaktorvolumen weniger Biogas und damit auch weniger nutzbares Methan erzeugt. Der Methananteil oder die Methanmenge haben Auswirkungen auf den wirtschaftlichen Betrieb eines Bioreaktors.

Aufgabe der Erfindung ist die Maximierung der umgesetzten Gärsubstratmenge und der dafür benötigten Verweildauer, sowie die Bildung einer größeren wirksamen Mischzone. Weitere Aufgabe ist es, ein Verfahren

2

zur Verfügung zu stellen, bei dem mehrere Rührwerke eine Mischzone erzeugen, wobei der dazu benötigte Energieaufwand minimiert und der aus der Mischzone resultierende Methanertrag optimiert wird.

Diese Aufgabe wird erfindungsgemäß dadurch gelöst, dass der Propellerdurchmesser, die Propellergeometrie und die Position der Propeller so gewählt sind, dass eine gemeinsame Mischzone des Mediums in dem Behälter erzeugbar ist, dass Messdaten zur Ermittlung der mittleren Geschwindigkeit und/oder der Viskosität des Mediums in der Mischzone erfasst werden und die Messdaten an eine Regeleinheit weitergeleitet werden, wobei durch die Regeleinheit Stellgrößen variierbar sind, welche den Leistungseintrag der Rührwerke in die Mischzone und/oder die Zusammensetzung des Behälterinhalts verändern. Ziel ist es, das Reaktionsvolumen zu vergrößern.

Mit Hilfe des Verfahrens ist es möglich, eine optimale Vermischung des Behälterinhalts, bei gleichzeitiger Minimierung des dazu notwendigen Energieeintrags, zu ermöglichen. Dabei erzeugt ein erstes Rührwerk einen Rührwerksstrahl. Ein zweites Rührwerk ist so positioniert, dass es den Strahl des ersten Rührwerks weitertransportiert und somit selbst einen Strahl erzeugt. Durch eine gezielte Positionierung der Rührwerke sowie dessen aufeinander abgestimmten Betrieb vergrößert sich das Mischvolumen zwischen den einzelnen Rührwerken. Erfindungsgemäß wird dazu auch von der Regeleinheit der Betrieb zusätzlicher Rührwerke geschaltet oder die Regeleinheit variiert die Menge und/oder die Zusammensetzung des zugeführten Substrats und/oder eines Rezirkulats.

Weiter wird die mittlere Geschwindigkeit der Strömung des Gärsubstrats in der Mischzone und/oder die Viskosität in der Mischzone erfasst. Als besonders vorteilhaft erweist es sich, wenn als Maßstab für einen optimalen Energieeintrag die mittlere Geschwindigkeit in der Mischzone herangezogen wird. Mit Hilfe dieser Größe wird bei dem erfindungsgemäßen Verfahren der Prozess energetisch optimiert. Werden die Rührwerke mit zu kleinen hydraulischen Leistungen betrieben, so ist die an einem Rührwerk vorhandene mittlere Austrittsgeschwindigkeit des Gärsubstrats in der Mischzone zu gering. Dies führt dazu, dass die Mischzone in nicht effektive Einzelzonen zerfällt. In diesen steigen die mittleren Geschwindigkeiten aufgrund der hohen lokalen Scherraten wieder an, was energetisch unwirtschaftlich ist. Werden die Rührwerke mit zu großen hydraulischen Leistungen betrieben, steigt die mittlere Geschwindigkeit in der Mischzone über das für ihre Bildung erforderliche Maß hinaus an, wodurch entweder der Energieeintrag und/oder der Methanertrag aus dem Biogas ungünstig beeinflusst wird. Zwischen diesen ungünstigen Betriebsbedingungen gibt es eine optimale Geschwindigkeit, die erfindungsgemäß als Regelgröße für einen optimalen Energieeintrag und Stoffumsatz im Behälter eingesetzt wird.

Die optimale Geschwindigkeit hängt von vielen Faktoren ab, beispielsweise der Position der Rührwerke und/oder der Zusammensetzung des Gärsubstrats. Somit ergeben sich für jeden Anwendungsfall spezifische optimale Geschwindigkeitswerte, bei denen eine Minimierung des Energieinhaltes durch optimalen Leistungs- und Schubeintrag der Rührwerke in eine gemeinsame Mischzone erreicht wird. Die Positionierung von Rührwerken wird so gestaltet, dass die Rührwerke nicht lokal begrenzt, sondern in Korrespondenz zueinander arbeiten.

Ein direkter Zusammenhang besteht bei strukturviskosen Medien zwischen der Geschwindigkeit und der Viskosität. Somit kann alternativ oder kumulativ zur Geschwindigkeit auch die Viskosität in Abhängigkeit von der Scherrate für die notwendige hydraulische Leistung hinsichtlich einer energetisch optimierten Durchmischung des Behälterinhalts herangezogen werden. Dazu erfolgt von einem oder mehreren Rührwerken eine direkte oder indirekte Leistungsermittlung auf der Basis elektrischer oder mechanischer Messdaten.

Der Zusammenhang zwischen Geschwindigkeit und Viskosität kann nach dem Ansatz von Ostwald-De-Waele beschrieben werden. Die örtliche Viskosität hängt von der lokal vorhandenen Scherrate ab. Diese steht wiederum im Zusammenhang mit der örtlichen Geschwindigkeit:

$$\eta = k \cdot \gamma^{m-1} = k \cdot \left(\frac{dv}{ds}\right)^{m-1}$$

mit $\eta$ : dynamische Viskosität,

$\gamma$ : Scherrate,

$\dfrac{dv}{ds}$ : Geschwindigkeitsgradient,

k, m: freie Parameter.

Nachdem Messdaten zur Ermittlung der mittleren Geschwindigkeit und/oder der Viskosität in der Mischzone erfasst wurden, werden die Messdaten an eine Regeleinheit weitergeleitet. Als Regeleinheit kann beispielsweise eine speicherprogrammierbare Steuerung eingesetzt werden.

Die Regeleinheit vergleicht die Messwerte mit anlagenspezifischen Sollwerten. Diese können die Geschwindigkeits- bzw. Viskositätswerte in der Mischzone sein, bei denen eine gemeinsame Mischzone bei einem minimalen Leistungseintrag der Rührwerke aufrechterhalten wird. Weiterhin sind die für die Methanerzeugung verwendeten Substrate, deren Mengen und Zusammenfassung, sowie deren Verweilzeit im Fermenter von Bedeutung. Die betriebsspezifischen Sollwerte hängen beispielsweise von der Größe des Behälters, der Art der Rührwerke und der Anordnung der Rührwerke zueinander ab. Sie werden für jeden Anwendungsfall festgelegt.

Bei einer besonders bevorzugten Ausführung der Erfindung wird als Stellgröße die Drehzahl der Rührwerke verwendet. Weichen Geschwindigkeit bzw. Viskosität von ihren Optimalwerten hinsichtlich Leistungseintrag oder Methangasertrag und/oder Methangasgehalt ab, so variiert die Regeleinheit die Drehzahl der Rührwerke. Ist die Geschwindigkeit zu groß bzw. die Viskosität zu gering, so wird die Drehzahl herabgesetzt. Bei zu kleiner Geschwindigkeit bzw. zu großer Viskosität erhöht die Regeleinheit die Drehzahl.

Der Leistungseintrag durch die Rührwerke kann auch dadurch variiert werden, dass weitere Rührwerke zugeschaltet werden. Die Zuschaltung liefert einen zusätzlichen Leistungseintrag und kann dadurch verhindern, dass die gemeinsame Mischzone in einzelne Kavernen zerfällt.

Die Regeleinheit kann auch Stellgrößen variieren, welche die Zusammensetzung des Behälterinhalts verändern. Dazu kann die Regeleinheit die Menge an zugeführtem Substrats erhöhen oder herabsetzen. Eine weitere Möglichkeit besteht darin, den Aufschluss des Gärsubstrats durch Enzymeinsatz zu verändern. Auch eine Verdünnung des Gärsubstrats mittels Gülle und/oder Rezirkulat sowie eine Veränderung der Fließeigenschaften des Gärsubstrats durch die Hinzufügung von chemischen oder biologischen Wirkmechanismen sind möglich. Dabei kann auch nur eine phasenweise Zuschaltung von einem oder mehreren Rührwerken erfolgen, beispielsweise bei einer zeitweiligen Änderung der Substratzusammensetzung oder der Gärsubstratzusammensetzung durch Rezirkulatzuführung.

Zur Bestimmung der mittleren Geschwindigkeit und der mittleren Viskosität in der Mischzone können unterschiedliche Messverfahren eingesetzt werden. Bei einer besonders vorteilhaften Variante der Erfindung wird die mittlere Geschwindigkeit in der Mischzone von Ultraschall-Sonden erfasst. Dies hat den Vorteil, dass es sich um ein kontaktfreies Messverfahren handelt. Bei der Messung der lokalen Geschwindigkeiten bleibt das Medium unbeeinflusst, da kein zusätzliches Hindernis in die Strömung eingebracht wird. Ein zusätzliches Hindernis würde eine Änderung der lokalen Scherrate nach sich ziehen. Aus diesem Grund werden vorzugsweise berührungslose Messverfahren eingesetzt.

Alternativ oder kumulativ zur Geschwindigkeitsmessung kann auch das Fließverhalten des Gärsubstrats erfasst werden. Bei einer besonders vorteilhaften Variante des Verfahrens werden die Messdaten zur Ermittlung der Viskosität in der Mischzone über die Leistungsaufnahme der Rührwerke erfasst. Dazu wird vorzugsweise ein Drehzahl geregeltes Rührwerk eingesetzt. Durch geregelte Drehzahlveränderung für Messzwecke kann der viskositätsabhängige Leistungsbeiwert als Funktion der Drehzahl und damit der Scherrate in der Rührwerksumgebung ermittelt werden. Die Messung, der am Rührwerk auftretenden Viskosität, erfolgt durch Kontrolle der Leistungsaufnahme des Rührwerks bei variabler Drehzahl. Durch Vergleich mit einer entsprechenden Wasserkurve kann die scherspannungsabhängige Viskosität des Mediums ermittelt werden. Es ist ein Ziel mit Hilfe von Auswertungen die Viskosität zu erfassen, um die hydraulische Leistung eines oder mehrerer Rührwerke bestimmen zu können.

In einer weiteren Ausführung werden im Betrieb Messdaten zur Ermittlung einer Schwimmschicht und deren Dicke erfasst. Diese Schwimmschichten bilden sich aus wenn Gasblasen an Partikeln haften, diese aufsteigen und sich an der Oberfläche zu durchgehenden Schichten verbinden. Eine derartige Schicht kann so stark anwachsen, dass ein Gasaustritt aus dem Gärsubstrat nicht mehr möglich ist. Die Entstehung dieser Schwimmschichten ist demnach zu unterbinden, wobei hierfür mindestens ein zusätzliches Rührwerk eingeschaltet wird, das eine die Schwimmschicht auflösende Turbulenz erzeugt. Durch die Turbulenz kann die Verbindung der Partikel zu einer Schicht unterbunden werden, sodass die Partikel wieder in das übrige Gärsubstrat eingerührt werden können.

Bei dem Verfahren werden vorzugsweise zylindrische Behälter zur Produktion von Biogas eingesetzt. Dabei erweist es sich als besonders günstig, wenn deren Verhältnis von Höhe zu Durchmesser kleiner als 0,5 ist. Alternativ können auch Ringbehälter bzw. Behälter mir umlaufender Strömung eingesetzt werden.

Weitere Merkmale und Vorteile der Erfindung ergeben sich aus der Beschreibung von Ausführungsbeispielen, anhand von Zeichnungen und aus den Zeichnungen selbst.

Dabei zeigt

Fig. 1 eine perspektivische Ansicht eines Behälters zur Biogasherstellung mit zwei Hauptrührwerken,

Fig. 2 eine schematische Darstellung der Regelung der Biogasherstellung,

Fig. 3 eine Simulation einer in einem Behälter mit drei Hauptrührwerken erzeugten Strömung.

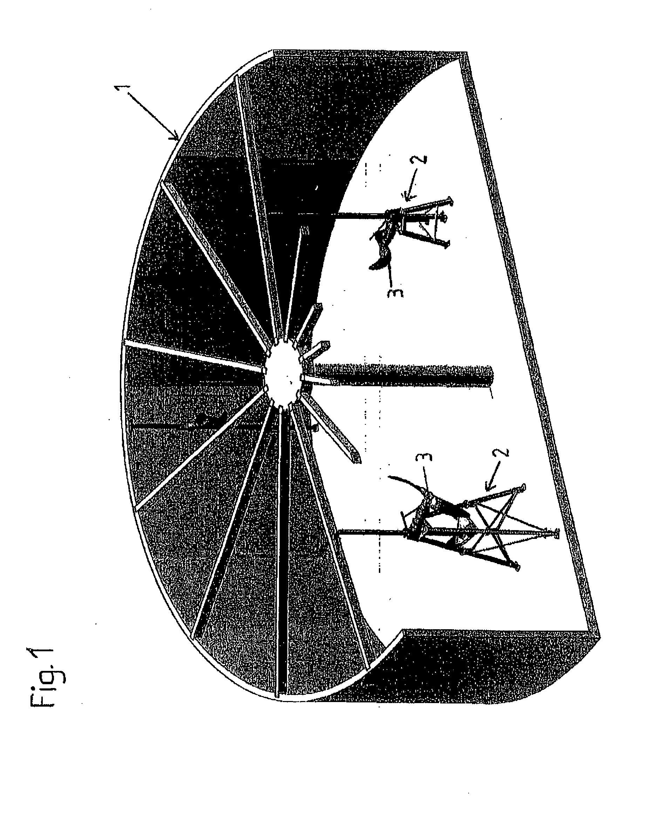

In Fig. 1 ist ein zylindrischer Behälter 1 zur Herstellung von Biogas dargestellt. Andere Behälterformen sind ebenfalls möglich. Das Verhältnis vom größten Durchmesser zur Höhe des Behälters ist kleiner 0,5. In dem Behälter 1 sind zwei Rührwerke 2 positioniert, deren Propeller 3 eine zu meist horizontale Strömungen des Gärsubstrats im Behälter 1 erzeugen. Die Rührwerke 2 beziehungsweise deren Propeller 3 sind in unterschiedlichen Höhen innerhalb des Behälters 1 angeordnet. Ein Ergänzungsrührwerk 11 wird bei Bedarf dazugeschaltet.

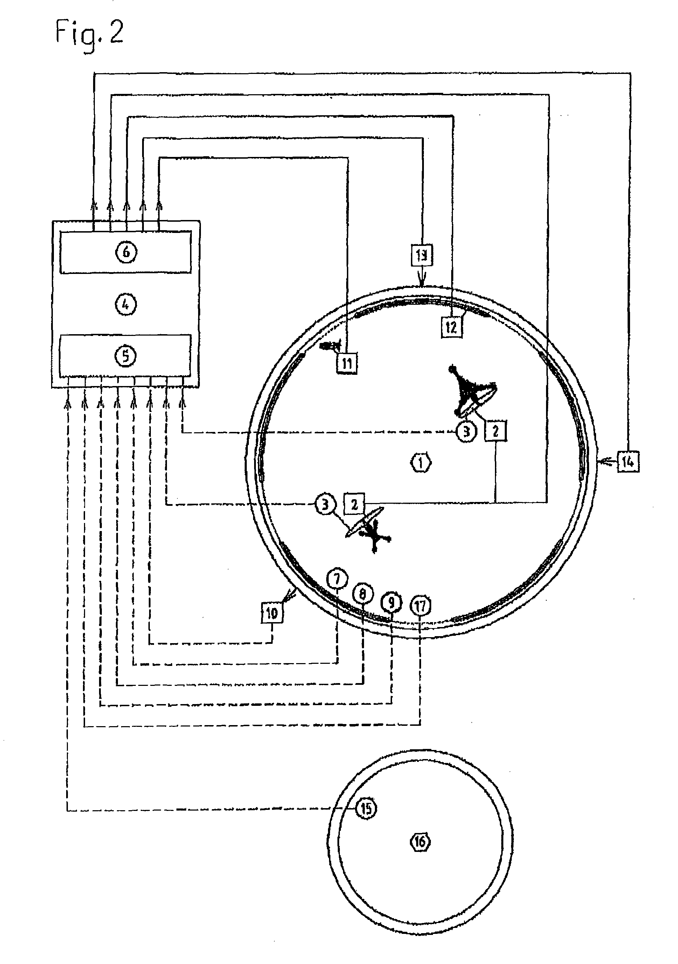

Fig. 2 zeigt eine schematisch dargestellte Regeleinheit 4 für ein Verfahren zur Biogasherstellung. Dabei kann es sich um eine speicherprogrammierbare Steuerung (SPS) oder ein anderes Regelsystem handeln.

Die Regeleinheit 4 verfügt über einen Signaleingangsbereich 5 und einen Signalausgangsbereich 6. An den Signaleingangsbereich 5 werden die Signale der Daten geleitet, die bei Messungen am Verfahren erfasst werden. In der Regeleinheit 4 werden Daten aus verschiedenen prozessüberwachenden Sensoren 7, 8, 9, 10, 15, 17 und den Rührwerken 2 verarbeitet. Der Signalausgangsbereich 6 steht in Wirkverbindung mit prozessbeeinflussenden Aggregaten. Prozessbeeinflussende Aggregate sind die Rührwerke 2, 11, eine Fermenterheizung 12, ein Aufgabesystem 13 und eine Rezirkulationseinheit 14. Diese werden so gesteuert, dass sich einzelne Prozessparameter mit dem Ziel eines maximalen Methanertrags optimieren lassen.

Folgende Sensoren können zur Prozessüberwachung eingesetzt werden: Mindestens ein Sensor zur Viskositätsmessung 7, ein oder mehreren Sensoren zur Fließgeschwindigkeitsmessung 8, mindestens ein Schwimmschichtdetektor 9, ein Gasmengenzähler 10, eine Einheit 15 zur Gärrestanalyse und mindestens eine Einheit 17 zur Fließverhaltenermittlung des Gärsubstrats.

Die Wirtschaftlichkeit einer Biogasanlage ist wesentlich von dem Eigenenergieverbrauch der Rührtechnik bzw. der Anlage abhängig. Dieser ist unter anderen abhängig von dem Fließverhalten des Gärsubstrates. Deshalb ist es eines der Hauptziele, eine möglichst geringe hydraulische Leistung für den Betrieb aufzuwenden.

Der Sensor 7 dient der Erfassung der Viskosität. Zur Ermittlung der Viskosität können auch Messdaten, welche über die Rührwerke 2 ermittelt werden, herangezogen werden. Alternativ oder ergänzend ist der Einsatz einer separaten Fließverhaltenermittlungseinheit 17 möglich. Die Fließverhaltensermittlung kann dabei an mehreren Stellen einzeln oder gleichzeitig erfolgen. Die Ermittlung des Fließverhaltens ist notwendig, um zu kritisches Fließverhalten im Behälter 1 hinsichtlich relevanter Prozessparameter sowie Schäden an allen im Prozess eingesetzten Rührwerken 2, 11 zu vermeiden und deren Eigenenergieverbrauch zu optimieren. Bei der Optimierung zwischen Gasertrag und Eigenenergieverbrauch ist die erzeugte Geschwindigkeit im Gärsubstrat von großer Bedeutung.

Der Sensor 8 wird zur Geschwindigkeitsmessung im Behälter 1 eingesetzt. Dabei kann die Geschwindigkeit mittels einer oder mehrerer Geschwindigkeitsermittlungen an unterschiedlichen Stellen erfolgen.

Mit einem Detektor 9 wird die Bildung einer Schwimmschicht überwacht. Da sich eine Schwimmschichtdecke nachteilig auf das Ausgasen des Biogases aus dem Gärsubstrat auswirkt, ist deren Entstehung zu vermeiden oder nach Entstehung möglichst früh zu zerstören. Dazu kann zum Beispiel ein Ergänzungsrührwerk 11 zugeschaltet und/oder ein oder mehrere Hauptrührwerke 2 können in ihrer Drehzahl variiert werden. Dadurch entsteht eine die Schwimmschicht auflösende Strömungsturbulenz.

In einer Biogasanlage ist die Methanerzeugung ein Hauptziel, weshalb bei dem Verfahren der erzeugte Gasmengenstrom und/oder Methanmengenstrom über einen Gasmengenzähler 10 erfasst wird. Sinkt die Methanmenge unter ein bestimmtes Niveau passt die Regeleinheit 4 die den Prozess beeinflussenden Aggregate an die Prozessbedingungen an. Ziel der Fermentation ist es, eine möglichst große Menge des Biogaspotentials des Substrates zu nutzen.

Die Gärreste werden in einem Gärrestelager 16 gesammelt. Die Ermittlung des Biogasrestpotentials im Gärrest erfolgt durch die Einheit 15 und ist eine weitere mögliche Eingangsgröße für die Regeleinheit 4 und die Regelung der Rührwerke 2, 11. Die Ermittlung des Biogasrestpotentials kann an unterschiedlichen Stellen der Anlage erfolgen. Ist im Gärrest ein bestimmtes Biogasrestpotential überschritten, passt die Regeleinheit 4 die prozessbeeinflussenden Aggregate 2, 11, 12, 13, 14 an die Prozessbedingungen an.

Grundsätzlich werden alle Daten aus dem Signaleingangsbereich 5 in der Regeleinheit 4 verarbeitet. Die Verarbeitung der Daten erfolgt auf Grundlage eines gespeicherten

Algorithmus. Dieser Algorithmus übernimmt die Aufgabe, aus den Eingangsgrößen die Werte für daraus bestimmte Regelgrößen zu ermitteln. Die ermittelten Regelgrößen werden dazu benutzt, um vom Signalausgangsbereich 6 die prozessbeeinflussenden Aggregate 2, 11, 12, 13, 14 zu regeln.

Von dem Signalausgangsbereich 6 gehen Signale zur Regelung unterschiedlicher Stellgrößen aus. Damit werden beispielsweise Rührwerke 2 angesteuert, wobei deren Drehzahl in Abhängigkeit der mittleren Geschwindigkeit im Mischvolumen und/oder der Viskosität im Mischvolumen geregelt werden kann. Wird eine bestimmte Grenzgeschwindigkeit des Gärsubstrats über- oder unterschritten, kann sich dies nachteilig auf die Strömungsbildung und damit auf den Prozess auswirken. Darüber hinaus kann es bei fehlender Bewegung an der Oberfläche des Gärsubstrats zur Ausbildung einer Schwimmschicht kommen. Weiterhin kann eine fehlende Bewegung dazu führen, dass das einzuspeisende Substrat oder das Gärsubstrat nur ungenügend im Behälter 1 verteilt werden.

Ein Ergänzungsrührwerk 11 kann bei der Zufuhr von neuem Substrat oder im Falle einer entstandenen Schwimmschicht dazugeschaltet oder geregelt werden. Die Heizung 12 führt bei der Einspeisung von neuem Substrat dem Behälter 1 Wärme zu. Das Substrat wird über das Aufgabesystem 13 zugeführt. Damit kann die Fütterungsmenge an die Prozessparameter angepasst werden. Eine Überfütterung des Behälters 1 mit Substrat würde sich nachteilig auf das Fließverhalten im Behälter 1 und damit auf die Methanproduktion auswirken. Verändert sich das Fließverhalten negativ, wird die Fütterungsmenge reduziert und/oder andere Regelgrößen, wie beispielsweise Geschwindigkeit oder Rezirkulatmenge, variiert.

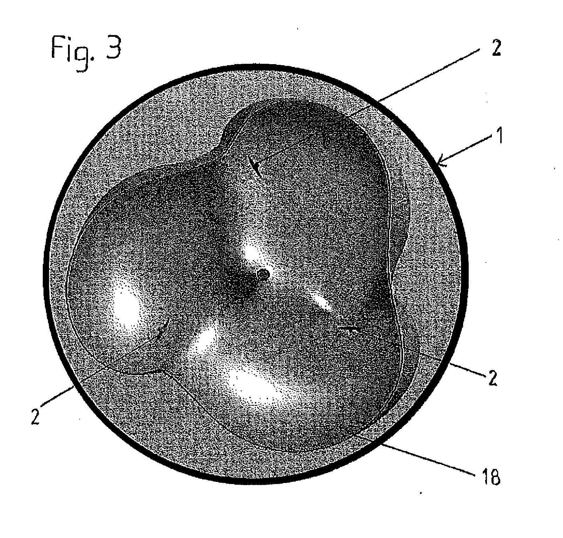

Bei zu geringen Fütterungsmengen steht nicht genügend Substrat zur Methanbildung zur Verfügung. Mit Hilfe des Gasmengenzähler 10 und/oder einer Analyse des Biogasrestpotentials im Gärrest wird dieser Zustand erkannt und eine Fütterung mit Substrat veranlasst. Das Verfahren verfügt über eine Rezirkulationseinheit 14, die im Falle eines zu kritischen Fließverhaltens, beispielsweise Rezirkulat zudosiert. Ziel ist die Veränderung des Fließverhaltens vom Gärsubstrat, um eine Strömungskorrespondenz zwischen den Rührwerken 2 zu ermöglichen. Diese Korrespondenz der Rührwerke gewährleistet ein möglichst optimales Reaktionsvolumen vom Gärsubstrat. Weiterhin sorgt eine Verbesserung des Fließverhaltens für einen besseren Transport der Schergeschwindigkeiten, wodurch eine vom Rührwerksstrahl gebildete Gärsubstrat-Kaverne größer wird. Optimal ist ein Ineinanderfließen solcher Kavernen, wie es als große Kaverne in Fig. 3 gezeigt ist. Dies sorgt für einen verbesserten Gasaustrag. Im Ergebnis verbessert sich der Biogas- und Methanertrag. Eine Verbesserung des Fließverhaltens ist auch durch Zudosierung von Enzymen, Spurenelementen oder anderen das Fließverhalten verändernden Substanzen möglich. Auf diese Weise werden ein höherer Substratumsatz und eine gesteigerte Biogasausbeute erzielt.

In Fig. 3 ist eine Simulation einer in einem Behälter 1 erzeugten Strömung mit drei horizontal angeordneten Rührwerken 2 dargestellt. Jedes Rührwerk 2 erzeugt eine Strombahn. Die Abstände der Rührwerken 2 zueinander sind so gewählt, dass eine gemeinsame Mischzone des Gärsubstrats in dem Behälter 1 erzeugt wird. Die Rührwerke 2 sind so positioniert, dass die jeweils vom benachbarten Rührwerk 2 erzeugte Strömung weitertransportiert wird und somit eine gemeinsame Mischzone entsteht. Die Grenzen der Mischzone sind durch Geschwindigkeitsgrenzwerte festgelegt. An der Oberfläche ist diese Geschwindigkeitsgrenze durch eine durchgezogene Linie 18 dargestellt.

**Patentansprüche**

1. Verfahren zur Herstellung von Biogas aus organischen Stoffen, wobei einem Behälter (1) Substrat mittels eines Aufgabesystems (13) zugeführt wird und in dem Behälter (1) mindestens zwei Rührwerke (2) angeordnet sind, deren Propeller (3) über Antriebe in Rotation versetzt werden, wobei die Propeller (3) in dem Behälter (1) zumeist horizontale Strömungen des Behälterinhalts erzeugen,
**dadurch gekennzeichnet, dass**
der Propellerdurchmesser, die Propellergeometrie und die Position der Propeller (3) so gewählt sind, dass eine gemeinsame Mischzone des Gärsubstrats in dem Behälter (1) erzeugbar ist, dass Messdaten zur Ermittlung der mittleren Geschwindigkeit und/oder der mittleren Viskosität des Gärsubstrats in der Mischzone erfasst werden und die Messdaten an eine Regeleinheit (4) weitergeleitet werden, wobei durch die Regeleinheit (4) Stellgrößen variierbar sind, welche den Leistungseintrag der Rührwerke (2) in die Mischzone und/oder die Zusammensetzung und/oder das Fließverhalten des Behälterinhalts verändern.

2. Verfahren nach Anspruch 1, **dadurch gekennzeichnet, dass** die Regeleinheit (4) als Stellgröße die Drehzahl von mindestens einem der Rührwerke (2) variiert.

3. Verfahren nach Anspruch 1 oder 2, **dadurch gekennzeichnet, dass** die Regeleinheit (4) mindestens ein zusätzliches Rührwerk (11) schaltet.

4. Verfahren nach einem der Ansprüche 1 bis 3, **dadurch gekennzeichnet, dass** die Regeleinheit (4) die Menge und/oder die Zusammensetzung des zugeführten Substrats und/oder eines Rezirkulats variiert und/oder die Fließeigenschaft des Gärsubstrats biochemisch oder physikalisch verändert.

5. Verfahren nach einem der Ansprüche 1 bis 4, **dadurch gekennzeichnet, dass** die mittlere Geschwindigkeit in der Mischzone von einer Ultraschall-Sonde erfasst wird.

6. Verfahren nach einem der Ansprüche 1 bis 5, **dadurch gekennzeichnet, dass** Messdaten zur Ermittlung der Viskosität über die Leistungsaufnahme von einem oder mehreren Rührwerken (2) erfasst werden.

7. Verfahren nach einem der Ansprüche 1 bis 6, **dadurch gekennzeichnet, dass** Messdaten zur Ermittlung des Fliessverhaltens über die Leistungsaufnahme von einem oder mehreren Rührwerken (2) erfasst werden.

8. Verfahren nach einem der Ansprüche 1 bis 7, **dadurch gekennzeichnet, dass** von einem oder mehreren Rührwerken (2) eine direkte oder indirekte Leistungsermittlung auf der Basis elektrischer oder mechanischer Messdaten erfolgt.

9. Verfahren nach einem der Ansprüche 1 bis 8, **dadurch gekennzeichnet, dass** Messdaten zur Ermittlung der Viskosität mit einem Viskosimeter erfasst werden.

10. Verfahren nach einem der Ansprüche 1 bis 9, **dadurch gekennzeichnet, dass** Messdaten zur Ermittlung einer Schwimmschicht und deren Dicke erfasst werden.

11. Verfahren nach Anspruch 10 **dadurch gekennzeichnet, dass** bei Auftreten einer Schwimmschicht mindestens ein zusätzliches Rührwerk (11) eingeschaltet wird, wobei dieses zusätzliche Rührwerk (11) eine die Schwimmschicht auflösende Turbulenz erzeugt.

12. Verfahren nach einem der Ansprüche 1 bis 11, **dadurch gekennzeichnet, dass** ein Behälter (1) eingesetzt wird, dessen Verhältnis von Höhe zu größtem Durchmesser kleiner als 0,5 ist.

13. Vorrichtung zur Herstellung von Biogas aus organischen Stoffen, wobei einem Behälter (1) Substrat mittels eines Aufgabesystems (13) zugeführt wird und in dem Behälter (1) mindestens zwei Rührwerke (2) angeordnet sind, deren Propeller (3) über Antriebe in Rotation versetzt werden, wobei die Propeller (3) in dem Behälter (1) zumeist horizontale Strömungen des Behälterinhalts erzeugen, **dadurch gekennzeichnet, dass** der Propellerdurchmesser, die Propellergeometrie und die Position der Propeller (3) so gewählt sind, dass eine gemeinsame Mischzone des Gärsubstrats in dem Behälter (1) erzeugbar ist, dass Messdaten zur Ermittlung der mittleren Geschwindigkeit und/oder der mittleren Viskosität des Gärsubstrats in der Mischzone erfasst werden und die Messdaten an eine Regeleinheit (4) weitergeleitet werden, wobei durch die Regeleinheit (4) Stellgrößen variierbar sind, welche den Leistungseintrag der Rührwerke (2) in die Mischzone und/oder die Zusammensetzung und/oder das Fließverhalten des Behälterinhalts verändern.

14. Vorrichtung nach Anspruch 13, **dadurch gekennzeichnet, dass** die Regeleinheit (4) als Stellgröße die Drehzahl von mindestens einem der Rührwerke (2) variiert.

15. Vorrichtung nach Anspruch 13 oder 14, **dadurch gekennzeichnet, dass** die Regeleinheit (4) mindestens ein zusätzliches Rührwerk (11) schaltet.

16. Vorrichtung nach einem der Ansprüche 13 bis 15, **dadurch gekennzeichnet, dass** die Regeleinheit (4) die Menge und/oder die Zusammensetzung des zugeführten Substrats und/oder eines Rezirkulats variiert und/oder die Fließeigenschaft des Gärsubstrats biochemisch oder physikalisch verändert.

17. Vorrichtung nach einem der Ansprüche 13 bis 16, **dadurch gekennzeichnet, dass** die mittlere Geschwindigkeit in der Mischzone von einer Ultraschall-Sonde erfasst wird.

18. Vorrichtung nach einem der Ansprüche 13 bis 17, **dadurch gekennzeichnet, dass** Messdaten zur Ermittlung der Viskosität über die Leistungsaufnahme von einem oder mehreren Rührwerken (2) erfasst werden.

19. Vorrichtung nach einem der Ansprüche 13 bis 18, **dadurch gekennzeichnet, dass** Messdaten zur Ermittlung des Fliessverhaltens über die Leistungsaufnahme von einem oder mehreren Rührwerken (2) erfasst werden.

20. Vorrichtung nach einem der Ansprüche 13 bis 19, **dadurch gekennzeichnet, dass** von einem oder mehreren Rührwerken (2) eine direkte oder indirekte Leistungsermittlung auf der Basis elektrischer oder mechanischer Messdaten erfolgt.

21. Vorrichtung nach einem der Ansprüche 13 bis 20, **dadurch gekennzeichnet, dass** Messdaten zur Ermittlung der Viskosität mit einem Viskosimeter erfasst werden.

22. Vorrichtung nach einem der Ansprüche 13 bis 21, **dadurch gekennzeichnet, dass** ein Behälter (1) eingesetzt wird, dessen Verhältnis von Höhe zu größtem Durchmesser kleiner als 0,5 ist.

## Claims

1. Method for producing biogas from organic substances, substrate being supplied to a container (1) by means of a feed system (13) and there being arranged in the container (1) at least two agitator mechanisms (2), the propellers (3) of which are set in rotation via drives, the propellers (3) generating in the container (1) mostly horizontal flows of the container contents, **characterized in that** the propeller diameter, the propeller geometry and the position of the propellers (3) are selected such that a common mixing zone of the fermentation substrate can be generated in the container (1), and **in that** measurement data for determining the average velocity and/or the average viscosity of the fermentation substrate in the mixing zone are detected and the measurement data are passed on to a regulating unit (4), the regulating unit (4) being capable of varying manipulated quantities which vary the introduction of power from the agitator mechanisms (2) into the mixing zone and/or the composition and/or the flow behaviour of the container contents.

2. Method according to Claim 1, **characterized in that** the regulating unit (4) varies the rotational speed of at least one of the agitator mechanisms (2) as a manipulated quantity.

3. Method according to Claim 1 or 2, **characterized in that** the regulating unit (4) switches at least one additional agitator mechanism (11).

4. Method according to one of Claims 1 to 3, **characterized in that** the regulating unit (4) varies the quantity and/or composition of the substrate supplied and/or of a recirculate and/or varies the flow property of the fermentation substrate biochemically or physically.

5. Method according to one of Claims 1 to 4, **characterized in that** the average velocity in the mixing zone is detected by an ultrasonic probe.

6. Method according to one of Claims 1 to 5, **characterized in that** measurement data for determining the viscosity are detected via the power consumption of one or more agitator mechanisms (2).

7. Method according to one of Claims 1 to 6, **characterized in that** measurement data for determining the flow behaviour are detected via the power consumption of one or more agitator mechanisms (2).

8. Method according to one of Claims 1 to 7, **characterized in that** direct or indirect power determination of one or more agitator mechanisms (2) takes place on the basis of electrical or mechanical measurement data.

9. Method according to one of Claims 1 to 8, **characterized in that** measurement data for determining the viscosity are detected by means of a viscosimeter.

10. Method according to one of Claims 1 to 9, **characterized in that** measurement data for determining a floating layer and its thickness are detected.

11. Method according to Claim 10, **characterized in that** at least one additional agitator mechanism (11) is switched on when a floating layer occurs, this additional agitator mechanism (11) generating turbulence which dissolves the floating layer.

12. Method according to one of Claims 1 to 11, **characterized in that** a container (1) is used, of which the ratio of height to largest diameter is lower than 0.5.

13. Appliance for producing biogas from organic substances, substrate being supplied to a container (1) by means of a feed system (13) and there being arranged in the container (1) at least two agitator mechanisms (2), the propellers (3) of which are set in rotation via drives, the propellers (3) generating in the container (1) mostly horizontal flows of the container contents, **characterized in that** the propeller diameter, the propeller geometry and the position of the propellers (3) are selected such that a common mixing zone of the fermentation substrate can be generated in the container (1), and **in that** measurement data for determining the average velocity and/or the average viscosity of the fermentation substrate in the mixing zone are detected and the measurement data are passed on to a regulating unit (4), the regulating unit (4) being capable of varying manipulated quantities which vary the introduction of power from the agitator mechanisms (2) into the mixing zone and/or the composition and/or the flow behaviour of the container contents.

14. Appliance according to Claim 13, **characterized in that** the regulating unit (4) varies the rotational speed of at least one of the agitator mechanisms (2) as a manipulated quantity.

15. Appliance according to Claim 13 or 14, **characterized in that** the regulating unit (4) switches at least one additional agitator mechanism (11).

16. Appliance according to one of Claims 13 to 15, **characterized in that** the regulating unit (4) varies the quantity and/or the composition of the substrate supplied and/or of a recirculate and/or varies the flow property of the fermentation substrate biochemically or physically.

17. Appliance according to one of Claims 13 to 16, **characterized in that** the average velocity in the mixing zone is detected by an ultrasonic probe.

18. Appliance according to one of Claims 13 to 17, **characterized in that** measurement data for determining the viscosity are detected via the power consumption of one or more agitator mechanisms (2).

19. Appliance according to one of Claims 13 to 18, **characterized in that** measurement data for determining the flow behaviour are detected via the power consumption of one or more agitator mechanisms (2).

20. Appliance according to one of Claims 13 to 19, **characterized in that** direct or indirect power determination of one or more agitator mechanisms (2) takes place on the basis of electrical or mechanical measurement data.

21. Appliance according to one of Claims 13 to 20, **characterized in that** measurement data for determining the viscosity are detected by means of a viscosimeter.

22. Appliance according to one of Claims 13 to 21, **characterized in that** a container (1) is used, of which the ratio of height to largest diameter is lower than 0.5.

**Revendications**

1. Procédé de production de biogaz à partir de substances organiques, du substrat étant acheminé à un récipient (1) au moyen d'un système de distribution (13) et au moins deux mécanismes d'agitation (2) étant disposés dans le récipient (1), dont les hélices (3) sont mises en rotation par le biais d'entraînements, les hélices (3) produisant dans le récipient (1) des écoulements essentiellement horizontaux du contenu du récipient, **caractérisé en ce que** le diamètre des hélices, la géométrie des hélices et la position des hélices (3) sont sélectionnés de telle sorte qu'une zone de mélange commune du substrat de fermentation puisse être produite dans le récipient (1), **en ce que** des données de mesure sont détectées dans la zone de mélange pour déterminer la vitesse moyenne et/ou la viscosité moyenne du substrat de fermentation et **en ce que** les données de mesure sont transmises à une unité de régulation (4), des grandeurs de commande pouvant être modifiées par l'unité de régulation (4), lesquelles modifient la puissance fournie par les mécanismes d'agitation (2) dans la zone de mélange et/ou la composition et/ou le comportement d'écoulement du contenu du récipient.

2. Procédé selon la revendication 1, **caractérisé en ce que** l'unité de régulation (4) fait varier en tant que grandeur de commande la vitesse de rotation d'au moins l'un des mécanismes d'agitation (2).

3. Procédé selon la revendication 1 ou 2, **caractérisé en ce que** l'unité de régulation (4) commute au moins un mécanisme d'agitation supplémentaire (11).

4. Procédé selon l'une quelconque des revendications 1 à 3, **caractérisé en ce que** l'unité de régulation (4) fait varier la quantité et/ou la composition du substrat acheminé et/ou d'un substrat recirculé et/ou la propriété d'écoulement du substrat de fermentation de manière biochimique ou physique.

5. Procédé selon l'une quelconque des revendications 1 à 4, **caractérisé en ce que** la vitesse moyenne dans la zone de mélange est détectée par une sonde à ultrasons.

6. Procédé selon l'une quelconque des revendications 1 à 5, **caractérisé en ce que** des données de mesure pour déterminer la viscosité sont déterminées par le biais de la consommation de puissance d'un ou de plusieurs mécanismes d'agitation (2).

7. Procédé selon l'une quelconque des revendications 1 à 6, **caractérisé en ce que** des données de mesure pour déterminer le comportement d'écoulement sont déterminées par le biais de la consommation de puissance d'un ou de plusieurs mécanismes d'agitation (2).

8. Procédé selon l'une quelconque des revendications 1 à 7, **caractérisé en ce qu'**une détermination de puissance directe ou indirecte est effectuée par un ou plusieurs mécanismes d'agitation (2) sur la base de données de mesure électriques ou mécaniques.

9. Procédé selon l'une quelconque des revendications 1 à 8, **caractérisé en ce que** des données de mesure pour déterminer la viscosité sont déterminées au moyen d'un viscosimètre.

10. Procédé selon l'une quelconque des revendications 1 à 9, **caractérisé en ce que** des données de mesure sont déterminées pour déterminer une couche flottante et son épaisseur.

11. Procédé selon la revendication 10, **caractérisé en ce qu'**en présence d'une couche flottante, au moins un mécanisme d'agitation supplémentaire (11) est branché, ce mécanisme d'agitation supplémentaire (11) générant une turbulence dissolvant la couche flottante.

12. Procédé selon l'une quelconque des revendications 1 à 11, **caractérisé en ce que** l'on utilise un récipient (1) dont le rapport hauteur à diamètre maximum est inférieur à 0,5.

13. Dispositif de fabrication de biogaz à partir de substances organiques, un substrat étant acheminé à un récipient (1) au moyen d'un système de distribution (13) et au moins deux mécanismes d'agitation (2) étant disposés dans le récipient (1), dont les hélices (3) sont mises en rotation par le biais d'entraînements, les hélices (3) produisant dans le récipient (1) des écoulements essentiellement horizontaux du contenu du récipient, **caractérisé en ce que** le diamètre des hélices, la géométrie des hélices et la position des hélices (3) sont sélectionnés de telle sorte qu'une zone de mélange commune du substrat de fermentation puisse être produite dans le récipient (1), **en ce que** des données de mesure sont détectées dans la zone de mélange pour déterminer la vitesse moyenne et/ou la viscosité moyenne du substrat de fermentation et **en ce que** les données de mesure sont transmises à une unité de régulation (4), des grandeurs de commande pouvant être modifiées par l'unité de régulation (4), lesquelles modifient la puissance fournie par les mécanismes d'agitation (2) dans la zone de mélange et/ou la composition et/ou le comportement d'écoulement du contenu du récipient.

14. Dispositif selon la revendication 13, **caractérisé en ce que** l'unité de régulation (4) fait varier, en tant que grandeur de commande, la vitesse de rotation d'au moins l'un des mécanismes d'agitation (2).

15. Dispositif selon la revendication 13 ou 14, **caractérisé en ce que** l'unité de régulation (4) commute au moins un mécanisme d'agitation supplémentaire (11).

**16.** Dispositif selon l'une quelconque des revendications 13 à 15, **caractérisé en ce que** l'unité de régulation (4) fait varier la quantité et/ou la composition du substrat acheminé et/ou d'un substrat recirculé et/ou la propriété d'écoulement du substrat de fermentation de manière biochimique ou physique.

**17.** Dispositif selon l'une quelconque des revendications 13 à 16, **caractérisé en ce que** la vitesse moyenne dans la zone de mélange est détectée par une sonde à ultrasons.

**18.** Dispositif selon l'une quelconque des revendications 13 à 17, **caractérisé en ce que** des données de mesure pour déterminer la viscosité sont déterminées par le biais de la consommation de puissance d'un ou de plusieurs mécanismes d'agitation (2).

**19.** Dispositif selon l'une quelconque des revendications 13 à 18, caractérisé en ce des données de mesure pour déterminer le comportement d'écoulement sont déterminées par le biais de la consommation de puissance d'un ou de plusieurs mécanismes d'agitation (2).

**20.** Dispositif selon l'une quelconque des revendications 13 à 19, **caractérisé en ce qu'**une détermination de puissance directe ou indirecte est effectuée par un ou plusieurs mécanismes d'agitation (2) sur la base de données de mesure électriques ou mécaniques.

**21.** Dispositif selon l'une quelconque des revendications 13 à 20, **caractérisé en ce que** des données de mesure pour déterminer la viscosité sont déterminées au moyen d'un viscosimètre.

**22.** Dispositif selon l'une quelconque des revendications 13 à 21, **caractérisé en ce que** l'on utilise un récipient (1) dont le rapport hauteur à diamètre maximum est inférieur à 0,5.

Fig. 1

EP 2 553 082 B1

EP 2 553 082 B1

Fig. 2

12

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19947339 A1
- WO 2008104320 A2
- DE 202007002835 U1